# EUROPEAN PATENT SPECIFICATION

(11) **EP 0 504 322 B1**
(45) Date of publication and mention of the grant of the patent: **14.02.1996**
(21) Application number: 91902841.5
(22) Date of filing: 07.12.1990
(51) Int. Cl.: C07D 317/42, C07C 255/10, C07C 55/32, C07C 69/63, C07C 211/15, C08G 63/68, C08G 69/42, C08G 73/10

(54) **PARTIALLY FLUORINATED COMPOUNDS AND POLYMERS**
TEILWEISE FLUORIERTE VERBINDUNGEN UND POLYMERE
COMPOSES ET POLYMERES PARTIELLEMENT FLUORURES

(30) Priority: 11.12.1989 US 450351
(43) Date of publication of application: 23.09.1992
(73) Proprietor: E.I. DU PONT DE NEMOURS AND COMPANY, Wilmington Delaware 19898 (US)
(72) Inventor: ANTON, Douglas, Robert, Claymont, DE 19703 (US); FARNHAM, William, Brown, Hockessin, DE 19707 (US); HUNG, Ming-Hong, Wilmington, DE 19810 (US); MCKINNEY, Ronald, James, Wilmington, DE 19803 (US); RESNICK, Paul, Raphael, Wilmington, DE 19805 (US)
(74) Representative: Jones, Alan John
(86) International application number: US9007052
(87) International publication number: WO9109025

(56) References cited:
- EP-A- 0 352 718
- FR-A- 1 404 744
- FR-A- 1 546 167
- FR-A- 2 126 340
- Chemical Abstracts, volume 60, no. 13, June 22 1964, (Columbus, Ohio, US), L.D.Moore: "Radical addition of perfluoroalkyl iodides and diiodides to allylmonomers"

## Description

### BACKGROUND OF THE INVENTION

### Field of the Invention

The present invention concerns partially fluorinated compounds which are useful as chemical intermediates or monomers. The invention further concerns partially fluorinated condensation polymers and dioxolanes.

### BACKGROUND OF THE INVENTION

Fluorinated intermediates are employed in the synthesis of a variety of chemical compounds, such as partially fluorinated polymers. Novel fluorinated intermediates and polymers derived therefrom are of significant interest to the chemical industry.

Partially fluorinated condensation polymers are known in the literature, see, e.g., I. L. Knunyants, C. Li, and V. V. Shokina, Russ. Chem. Rev., 32:461 (1963), and the references described therein. These polymers generally have been made to obtain materials with improved thermo-oxidative stability and solvent resistance, as compared to nonfluorinated polymers.

Many partially fluorinated condensation polymers have been made with perfluoro diacids, HOOC-(CF₂)ₙ-COOH, as one component of the polymer, see, e.g., B. S. Marks & G. C. Schweiker, J. Polymer Sci. 43:229 (1960) wherein the synthesis of partially fluorinated polyamides containing perfluoro diacid units is disclosed. The polymers made from such perfluoro diacids, however, suffer from problems associated with the extreme hydrolytic instability of the perfluoroacyl functional groups. The strongly electron-withdrawing perfluoroalkyl group situated next to the carbonyl of the perfluoroacyl group activates the carbonyl to hydrolysis. It has been found that many of the resulting polymers hydrolyze in moist air.

P. Johncock, S. P. Barnett, & P. A. Rickard, J. Polymer Sci./Polymer Chem. 24:2033 (1986) disclose the synthesis of a diacid wherein the fluoroalkyl chain is separated from the acyl group by a methylene spacer, HOOC-CH₂-(CF₂)₃-CH₂-COOH. Although polyesters made with this diacid are more hydrolytically stable than those made from perfluoro diacids, these compositions still hydrolyze under mild conditions. This acid also suffers from the ready loss of two moles of hydrogen fluoride from the molecule.

P. L. Coe., N. E. Milner & J. A. Smith, J. Chem. Soc./Perkin I 654 (1975) disclose use of perfluoroalkylcopper compounds to form certain polyfluoroalkyl-substituted acids and alcohols. These compounds are said to be of practical interest as surfactants. In one reaction, (1E,6E)-3,3,4,4,5,5-hexaf1uoro-1,7-di-iodohepta-1,6-diene was reacted with copper(I) cyanide. Hydrolysis of the resulting unsaturated dinitrile yielded a diacid that was hydrogenated to give HOOC-CH₂CH₂-(CF₂)₃-CH₂CH₂-COOH.

US-A-3 496 215 discloses hydrocyanation of unsaturated compounds using as a catalyst a compound of the structure Ni(PXYZ)₄ wherein X is OR; Y and Z are R or OR; and R is an alkyl or aryl radical of up to 18 carbon atoms. US-A-3 496 217 discloses hydrocyanation of nonconjugated ethylenically unsaturated organic compounds using certain nickel complexes as a catalyst and certain zinc-containing compounds as promoters US-A-3 496 218 discloses hydrocyanation of nonconjugated ethylenically unsaturated organic compounds using certain nickel complexes as catalysts and certain organoboron compounds as promoters.

C. A. Tolman et al., Adv. in Catalysis, 33:1 (1985) disclose homogeneous nickel-catalyzed olefin hydrocyanation, as well as unpromoted hydrocyanations of monoolefins and hydrocyanations promoted with Lewis acids.

The synthesis of compositions of the present invention starts with bis-2,2-trifluoromethyl-4,5-difluorodioxole, 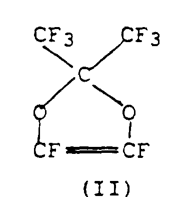 This dioxole was claimed by Resnick in US-A-3 865 845.

### SUMMARY OF THE INVENTION

The present invention provides dioxolanes of the formula 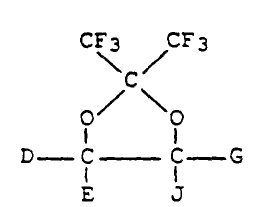 wherein D, E, G and J are:

| D | E | G | J |
|---|---|---|---|
| Cl | I | Cl | Cl |
| Br | H | Br | Cl |
| Br | Cl | Br | Cl |
| F | F | Cl | Cl |
| F | I | F | I |
| F | I | F | Br |
| F | I | F | Cl |
| F | I | F | F |
| F | Br | F | F |
| F | CH₂CH₂I | F | CH₂CH₂I |
| F | CH₂CH₂I | F | F |
| F | CH₂CH₂I | F | Cl |
| F | CH₂CH₂I | F | Br |
| F | CH=CH₂ | F | CH=CH₂ |
| F | CH=CH₂ | F | F |
| F | CH=CH₂ | F | Cl |
| F | CH=CH₂ | F | Br |
| F | COOH | F | F |
| F | COOH | F | Cl |
| F | COOH | F | Br |
| F | COCl | F | F |
| F | COF | F | F |
| F | CH₂CH₂CH₂NH₂ | F | CH₂CH₂CH₂NH₂ |
| H | CH=CH₂ | H | CH=CH₂ |
| H | CH₂CH₂CN | H | CH₂CH₂CN |
| H | CH₂CH₂CH₂NH₂ | H | CH₂CH₂CH₂NH₂ |

### DETAILED DESCRIPTION OF THE INVENTION

The halogen addition reactions of Examples 1-4 and 21, 22, 23 and 24 may be conducted at conditions of time and temperature sufficiently strenuous to result in acceptable conversion of the starting materials, but not strenuous enough to result in low yield of the desired product. The mole ratio of the halogen to the dioxole may be 0.6 to 1.4, preferably 1:1.

The ethylene addition reactions of Examples 5, 7, 8, and 14, may be started at room temperature and heated gradually or rapidly to the final reaction temperature of 200-250°, preferably 220° as in the Examples. The time at temperature may be 0.5 to 24 hours, depending on the temperature chosen. Longer time is needed at lower temperature.

The dehydrohalogenation reactions of Examples 6, 9, 10 and 15 use a concentrated strong base, preferably KOH or NaOH, at 30-60° for 10-24 hours. Preferably a quaternary ammonium salt is added to a phase transfer catalyst.

The oxidation of the vinyl compounds to carboxyl compounds, Examples 11, 12 and 18, uses a permanganate salt at 25-75°, continuing until the exothermic reaction is finished. Inorganic acid or a quaternary ammonium salt is an optional additive.

The polymerizations may be carried out in any of the methods known for polymerization of tetrafluoroethylene, such as aqueous, nonaqueous, and two-phase. The temperature is selected to match the decomposition rate of the initiator chosen, which should be nontelogenic.

The copolymers, which contain at least 1 weight % of the vinyl compound of this invention, are useful for electrical insulation, among other uses. The compounds with five-membered rings are useful as monomers or intermediates to monomers.

In the following examples, temperatures are in degrees Celsius.

The present invention provides partially fluorinated compounds which are useful as chemical intermediates. The unsaturated nitrile compounds are useful for the preparation of unsaturated acids, amines and esters. The unsaturated nitriles and esters are intermediates for the production of dinitriles and esternitriles of the present invention. The dinitrile compounds are useful as intermediates for the production of diacids, diesters, diisocyanates, and diamines. The esternitrile compounds can be also converted to esteramines. These difunctional species are useful for the synthesis of partially fluorinated condensation polymers. Polymers prepared with compounds of the invention demonstrate improved hydrolytic stability and altered dielectric constants.

The compounds and polymers of the present invention are described in the following examples.

### EXAMPLE 1

### Preparation of Bis-2,2-trifluoromethyl-4,5-difluoro-4,5-diiodo-1,3-dioxolane (I)

A mixture of 54 g. iodine and 52 g. 2,2-bistrifluoromethyl-4,5-difluorodioxole, (II) was heated in a tube for four hours at 100°. The product was filtered and distilled to give 67.2 g. (I), boiling at 44-45° at 10 torr. The ¹⁹F NMR spectrum [trans isomer -26.7 (2F), -79.0 (6F); cis isomer -39.5 (2F), -77.8 (3F), -79.0 (3F)] and the infrared spectrum were consistent with structure (I).

### EXAMPLE 2

### Preparation of Bis-2,2-trifluoromethyl-4-bromo-5-iodo-4,5-difluoro-1,3-dioxolane (III)

(II), 98 g., was slowly added to a mixture of 83 g. iodine bromide and 100 ml. 1,1,2-trichloro-1,2,2-trifluoroethane. The reaction mixture was treated with aqueous sodium bisulfite and distilled to give 97.8 g. (III), boiling at 64° at 50 torr. The ¹⁹F NMR spectrum [trans isomer -32.5 (1F), -33.9 (1F), -79.0 and -79.8 (6F); cis isomer -38.0 (1F), -53.1 (1F), -79.0 and 79.8 (6F)] and the infrared spectrum were consistent with structure (III).

### EXAMPLE 3

### Preparation of Bis-2,2-trifluoromethyl-4-chloro-5-iodo-4,5-difluoro-1,3-dioxolane (IV)

A mixture of 49 g. iodine monochloride and 48.8 g. (II) was heated in a tube at 100° for four hours. The product was treated with aqueous sodium bisulfite and distilled to give 49.2 g. (IV) boiling at 50° at 50 torr. The ¹⁹F NMR spectrum [trans isomer -36.4 (1F), -40.0 (1F), -80.2 (6F); cis isomer -38.3 (1F), -61.7 (1F), -79.5 (3F), -80.2 (3F)] and the infrared spectrum were consistent with structure (IV).

### EXAMPLE 4

### Preparation of Bis-2,2-trifluoromethyl-4,4,5-trifluoro-5-iodo-1,3-dioxolane (V)

Iodine pentafluoride, 6.1 g., was added to a mixture of 12.8 g. iodine and 50 ml. 1,1,2-trichloro-1,2,2-trifluoroethane. After stirring 30.5 g. (II) was added, stirred at room temperature for 16 hours and heated to reflux for 2.5 hours. After cooling the reaction mixture was treated with aqueous sodium bisulfite and distilled to give 2.1 g. (V) boiling at 40° at 100 torr. The ¹⁹F NMR spectrum [-46.7 (1F), -60.4 and -87.3 AB (2F) J_{AB}=127 Hz., -80.9 (6F)] was consistent with structure (V).

### EXAMPLE 5

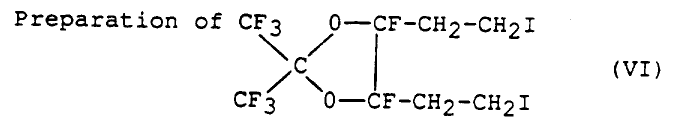

A mixture of 66.0 g. (I) and 15 g. of ethylene was heated at 150° for 30 minutes, 200° for 30 minutes and 220° for 10 hours. The product was distilled to give 47.0 g. (VI) boiling at 105° at 4 torr. The ¹⁹F NMR spectrum [trans isomer -79.4 (6F), -106.8 (2F); cis isomer -79.4 (3F), -80.5 (3F), -109.0 (2F)], ¹H NMR spectrum [3.6 (4H), 2.95 (4H)] and infrared spectrum were consistent with structure (VI).

### EXAMPLE 6

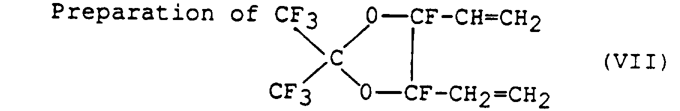

A mixture of 100 ml. 50% aqueous potassium hydroxide, 0.9 g. bis(2-hydroxypropyl)benzyldodecylammonium chloride, (VIII), and 46.2 g. (VI) was heated at 50° for 16 hours. The reaction mixture was distilled and the lower layer redistilled to give 19.1 g. (VII) boiling at 130°. The infrared spectrum, ¹⁹F NMR spectrum [-79.6 (6F), -108.2 (2F)] and ¹H NMR spectrum were consistent with structure (VII).

### EXAMPLE 7

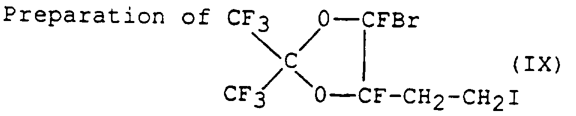

A mixture of 95.0 g. (III) and 10 g. ethylene was heated at 220° for four hours. The product was distilled to give 87.1 g. (IX) boiling at 86° at 20 torr. The infrared and NMR spectra [1H 3.7 (2H), 3.2 (2H); ¹⁹F trans isomer -53.0 (1F), -79.4 (3F), -80.0 (3F), -94.5 (1F); cis isomer -55.9 (1F), -79.4 (3F),-80.0 (3F), -105.6 (1F) were consistent with structure (IX).

### EXAMPLE 8

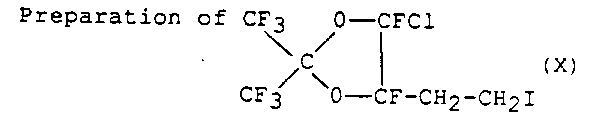

A mixture of 28.5 g. (IV), 51.9 g. 1,1,2-trichloro-1.2.2-trifluoroethane and 10 g. ethylene was heated at 150° for 30 minutes, 200° for 30 minutes and 220° for 10 hours. The product was distilled to give 29.8 g. (X) boiling at 80-82° at 25 torr. The infrared and NMR spectra [¹H 3.5 (2H), 3.0 (2H) ; ¹⁹F trans isomer -59.2 (1F), -80.0 (3F), -80.5 (3F), -99.2 (1F); cis isomer -63.9 (1F), -80.0 (3F), -80.5 (3F), -105.8 (1F)] were consistent with structure (X).

### EXAMPLE 9

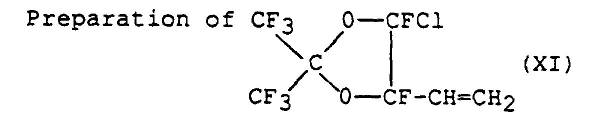

A mixture of 50 ml. 50% aqueous sodium hydroxide, 0.6 g. (VIII), and 29.8 g. (X) was heated at 45-50° for 16 hours. The reaction mixture was distilled to give 19.1 g. (XI) as a lower layer, b.p. 108°. The ¹H [complex peaks 5.2 to 5.7] and ¹⁹F NMR [trans isomer -57.9 (1F), -81.0 (3F), -81.5 (3F), -102.2 (1F); cis isomer -66.5 (1F), -81.0 (3F), -81.5 (3F), -106.7 (1F)] were consistent with structure (XI).

### EXAMPLE 10

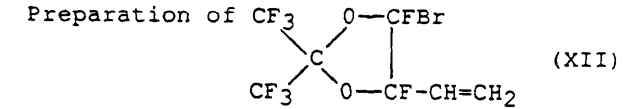

A mixture of 103 ml. 50% aqueous sodium hydroxide, 2.5 ml. of 60% (VIII) and 87.1 g. (IX) was heated at 50° for 16 hours. The mixture was distilled to give a liquid with two layers. The lower layer was redistilled to give 40.7 g. (XII) boiling at 120-122°. The infrared and NMR spectra [¹H complex peaks 5.5 to 6.1; ¹⁹F trans isomer -50.8 (1F), -79.7 (3F), -80.3 (3F), -97.1 (1F); cis isomer -57.3 (1F), -79.7 (3F), -80.3 (3F), -105.0 (1F)] were consistent with structure (XII).

### EXAMPLE 11

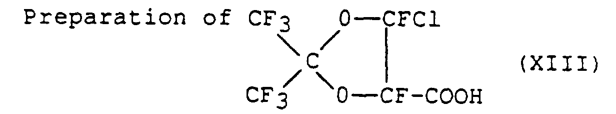

A cooled mixture of 134 ml. water, 27 g. conc. sulfuric acid and 32 g. potassium permanganate was stirred and 16.6 g. (XI) added slowly. The mixture was heated slowly and an exothermic reaction took place at 50°. After cooling the potassium permanganate and manganese dioxide were destroyed by reaction with aqueous sodium sulfite. The lower layer was separated and the upper layer extracted four times with 75 ml. ether. The combined ethereal extracts and lower layer were combined, dried with calcium chloride and distilled to give 7.3 g. (XIII) boiling at 107° at 40 torr. The infrared and NMR spectra [¹H 11.15; ¹⁹F trans isomer -58.0 (1F), -80.3 (3F), -81.1 (3F), -102.1 (1F); cis isomer -64.2 (1F), -80.3 (3F), -81.1 (3F), -107.8 (1F)] were consistent with structure (XIII).

### EXAMPLE 12

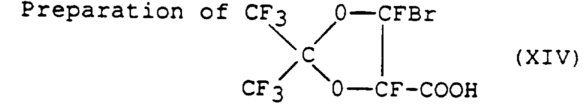

A mixture of 177 ml. water, 74.3 g. potassium permanganate and 3.54 g. methyltrioctylammonium chloride was stirred and 40.7 g. (XII) added in 45 minutes. The temperature rose to 52° and was maintained at 4-45° with external cooling for 5 hours. After heating to 70° followed by immediate cooling the mixture was allowed to stand at room temperature for 16 hours. After acidification with sulfuric acid the excess potassium permanganate and manganese dioxide were destroyed with aqueous sodium bisulfite. The lower layer was separated and the upper layer extracted twice with 100 ml. ether. The extracts were combined with the lower level, dried with anhydrous magnesium sulfate and distilled to give 22.4 g. (XIV) boiling at 99° at 20 torr. The ¹⁹F NMR spectrum [trans isomer -53.8 (1F), -80.5 (6F), -98.0 (1F); cis isomer -58.5 (1F), -79.7 (3F), -80.5 (3F), -107.7 (1F)] was consistent with structure (XIV).

### EXAMPLE 13

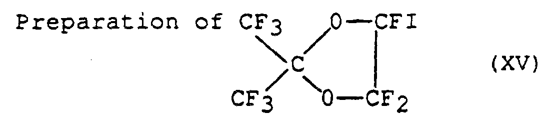

A mixture of 48.8 g. (II), 45 g. yellow mercuric oxide, 127 g. iodine, 12 g. anhydrous hydrogen fluoride, 0.1 g. phenothiazine and 0.055 g. hydroquinone was heated in a stainless steel tube at 50° for two hours, the temperature raised to 125° over a two hour period and at 125° for three hours. The reaction mixture was poured into ice water and the lower organic layer distilled to give 50.0 g., 64%, (XV) boiling at 74-76°. The ¹⁹F NMR spectrum was consistent with structure (XV).

### EXAMPLE 14

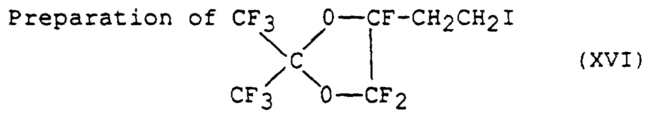

A mixture of 11.7 g. (XV) and 5.0 g. ethylene was heated in a stainless steel tube at 220° for 10 hours. The liquid product was distilled to give 7.0 g., 56%, (XVI) boiling at 95° at 100 torr. The NMR spectra [¹H 2.90 (2H), 3.43 (2H); ¹⁹F -77.8 (1F), -80.9 (3F), -81.6 (3F), -88.3 (1F), -110.0 (1F)] are consistent with structure (XVI).

### EXAMPLE 15

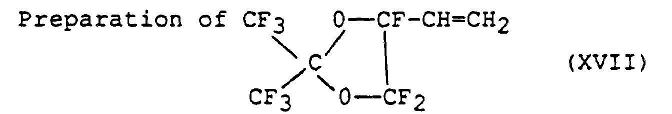

A mixture of 16.7 g. (XVI), 20 ml. 10M potassium hydroxide and 0.86 g. (VIII) was stirred at room temperature and monitored by gas chromatography until no more (XVI) remained. The lower organic layer was separated, washed with water, dilute hydrochloric acid and distilled to give 9.5 g., 82%, (XVII) boiling at 70-72°. The NMR spectra [¹H 5.40-5.90 complex; ¹⁹F -75.7 (1F), -82.0 (3F), -82.6 (3F), -90.3 (1F), -112.8 (1F)] are consistent with structure (XVII).

### EXAMPLE 16

### Copolymerization of (XVII) and Tetrafluoroethylene

A mixture of 5.0 g. (XVII), 30 g. of 1,1,2-trichloro-1,2,2-trifluorethane, 0.05 g. "Percadox" 16N, 1.0 g. tetrafluoroethylene and 750 psi nitrogen was heated at 60° for 5 hours. The solvent was evaporated to yield 0.5 g. polymer containing 84.1 mole percent tetrafluoroethylene and 15.9 mole percent (XVII) as determined by ¹⁹F NMR spectroscopy.

### EXAMPLE 17

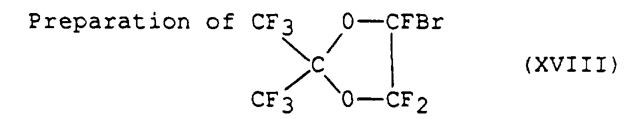

A mixture of 97.6 g. (II), 90 g. yellow mercuric oxide, 160 g. bromine, 36 g. anhydrous hydrogen fluoride, 0.2 g. phenothiazine and 0.11 g. hydroquinone was heated at 50° for two hours, the temperature raised to 120° over a two hour period and held at 120° for three hours. After filtration and washing with a saturated solution of sodium thiosulfate the organic layer was distilled to give 13.5 g., (10%), (XVIII) boiling at 60° and 66 g., (41%), bis-2,2-trifluoromethyl-4,5-dibromo-4,5-difluoro-1,3-dioxolane. The ¹⁹F NMR spectrum [-54.5 (1F), -67.1 (1F), -81.6 (6F), -85.6 (1F)] is consistent with structure (XVIII).

### EXAMPLE 18

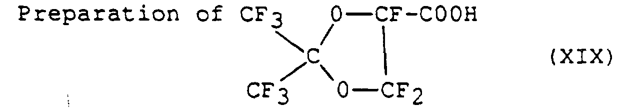

(XVII), 29.0 g, was slowly added to a mixture of 50 g. concentrated sulfuric acid, 100 ml. water and 31.6 g. potassium permanganate. After stirring for 6 hours at room temperature the reaction mixture was extracted with ether, the organic layer washed with water and distilled to give 8.0 g., 26%, (XIX), boiling at 71-71° [?] at 12-15 Torr. The ¹⁹F NMR spectrum [-74.8 (1F), -80.6 (6F), -84.5 (1F), -113.7 (1F)] is consistent with structure (XIX).

### EXAMPLE 19

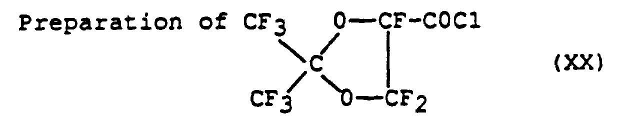

Thionyl chloride, 14.3 g., was slowly added to a mixture of 30 g. (XIX) and 3.16 g. pyridine and slowly heated to 85-90°. After heating at this temperature for 1.5 hours the volatile product was codistilled with thionyl chloride and was separated in a separatory funnel. (XX), 15.0 g., boiled at 79-80°. The ¹⁹F NMR spectrum [-74.5 (1F), -81.3 (3F), -81.8 (3F), -84.0 (1F), -109.1 (1F)] is consistent with structure (XX).

### EXAMPLE 20

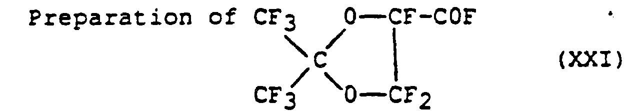

A mixture of 6.53 g. (XX), 4.06 g. potassium fluoride and 10 ml. tetramethylene sulfone was slowly heated to 140°. (XXI), 4.5 g., 72.6%, was obtained as a clear colorless oil boiling at approximately 50°. The ¹⁹F NMR spectrum [+23.9 (1F), -74.4 (1F), -81.2 (6F), -84.1 (1F), -113.3 (1F)] is consistent with structure (XXI).

### EXAMPLE 21

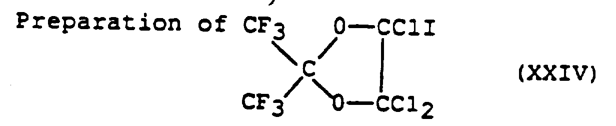

A mixture of 27.7 g. 2,2-bis-trifluoromethyl-4,5-dichloro-1,3-dioxole and 18 g. iodine monochloride was irradiated with a sun lamp. The reaction mixture was washed with aqueous sodium bisulfite and the organic layer distilled to give 6.5 g. (XXIV) boiling at 61-64° at 10 torr. which slowly turned purple on standing. The infrared and ¹⁹F NMR spectra [-76.4 complex] are consistent with structure (XXIV).

### EXAMPLE 22

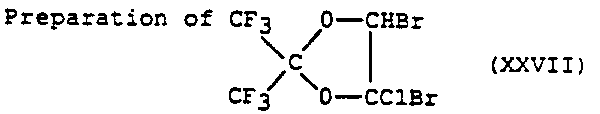

Bromine was slowly added to 36.3 g. (XXV) while irradiating with a sun lamp. The excess bromine was destroyed with aqueous sodium bisulfite and the organic layer was washed with water and distilled to give 51.9 g. (XXVII) boiling at 63° at 20 torr. The infrared and NMR [¹H 7.24 (trans), 6.98 (cis); ¹⁹F -77.7, -78.6] spectra are consistent with the cis and trans isomers of structure (XXVII).

### EXAMPLE 23

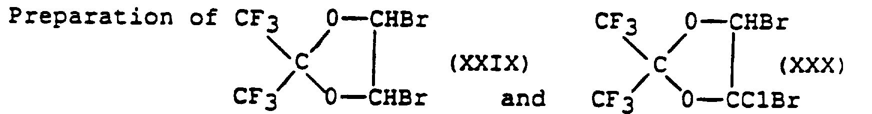

A 15.6 g. mixture of 2,2-bis-trifluoromethyl-1,3-dioxole and 2,2-bis-trifluoromethyl-4-chloro-1,3-dioxole was irradiated with a sun lamp. Bromine was added slowly and after 25 minutes the excess bromine was destroyed with aqueous sodium bisulfite. The organic layer was washed with water, dried with calcium chloride and distilled to give (XXIX) boiling at 69° at 50 torr and (XXX) boiling at 78° at 50 torr. The NMR spectra were consistent with these structures. [(XXIX) ¹H 7.02; ¹⁹F -77.7; (XXX) ¹H 7.29; ¹⁹F -77.6 (3F) -78.4 (3F)]

### EXAMPLE 24

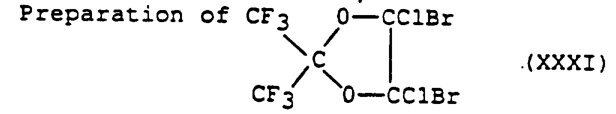

The procedure of Example 27 was followed and 65.0 g. 2,2-bis-trifluoromethyl-4,5-dichloro-1,3-dioxole was brominated to give 93.9 g. cis/trans (XXXI) boiling at 97° at 50 torr. The infrared and NMR spectra [¹⁹F trans -76.5, cis -76.1 (3F), -76.8 (3F)] are consistent with structure (XXXI).

### EXAMPLE 25

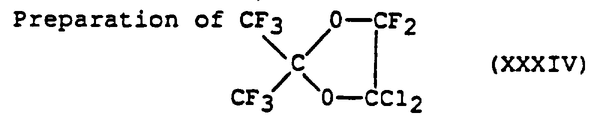

A glass ampoule containing 8.8 g. (XXXIII) and 8.4 g. chlorine was sealed and irradiated with a sun lamp for 16 hours. The tube was opened, washed with aqueous sodium bisulfite, water and distilled to give 4.7 g. (XXXIV) boiling at 86°. The infrared and NMR [¹⁹F -71.1 (2F), -80.8 (6F)] spectra were consistent with structure (XXXIV).

### EXAMPLE 26

### Copolymerization of Tetrafluoroethylene and (VII)

A mixture of 12.1 g. (VII), 0.2 g. bis(4-t-butylcyclohexyl) peroxydicarbonate, (XXXV), 45 g. tetrafluoroethylene and 100 ml. 1,1,2-trichloro-1,2,2-trifluoroethane was heated at 60° for one hour. 70° for one hour and 80° for one hour. The reaction mixture was evaporated to give a solid residue which was washed with an acetone/water mixture and then acetone. It was dried to yield 7.3 g. of a white solid whose infrared spectrum was consistent with a copolymer of (VII) and tetrafluoroethylene.

### EXAMPLE 27

### Copolymerization of Tetrafluoroethylene and (XI)

A mixture of 200 ml. of water, 1.0 g. ammonium perfluorononanoate, 0.2 g. ammonium persulfate, 10 g. (XI) and 45 g. tetrafluoroethylene was heated at 80° for one hour and 90° for two hours. The reaction mixture, which contained a liquid and solid, was stirred vigorously with a few drops of triethylenetetramine to give additional copolymer and filtered.

### EXAMPLE 28

### Preparation of 2,2-Bis(Trifluoromethyl)-4,5-Di(3-Aminopropyl)-4,5-Difluoro-1,3-Dioxolane

In a 1400 ml Hastelloy-C Shaker tube was charged the 2,2-bis(trifluoromethyl)-4,5-di(2-cyanoethyl)-4,5-difluoro-1,3-dioxolane (56.3 g, 0.16 mole), tetrahydrofuran (320 ml) and Raney-Cobalt catalyst (14.4 g). The tube was sealed and cool-evacuated. Ammonia (64 g, 3.765 mole) was transferred into the tube and the tube was pressurized with hydrogen gas to 500 psi. The tube was then heated to l10°C and the hydrogen pressure was adjusted to 1500 psi. The heating was continued for 18 hrs at this temperature. After the tube was cooled, the product mixture was filtered to remove the catalyst, and solvent was removed in vacuo. The residue was distilled to give the desired product 46 g (80% yield) as a clear, colorless, viscous liquid, Bp. 80°C/0.06 mmHg. H-1 NMR (CDCl₃): 3.02 (m, 4H), 2.30 (m, 4H), 1.97 (m, 4H), 1.50 (s, br, -NH₂); F-19 NMR (CDCl₃): -80.5 (m, 6F), [-108.8 (m, br, trans), -110.6 (m, br cis) (2F total)].

### EXAMPLE 29

### Preparation of 2,2-Bis(Trifluoromethyl)-4,5-Divinyl-1,3-Dioxolane

2,3-Divinylethylene oxide was prepared according to E. L. Stogryn et al., J. Org. Chem., 29, 1275 (1964). This compound (60 g, 0.625 mole) was mixed with d-limonene (2 g), tetra-n-butylammonium bromide (0.6 g), water (0.6 g) and hexafluoroacetone (108 g, 0.65 mole) in a 360 ml Shaker tube. The tube was sealed and was heated at 80°C/1 h4, 100°C/1hr and 120°C/6 hrs. The product mixture unloaded from the tube was distilled to give the desired product 30 g as a clear, colorless liquid. Bp. 50°C/30 mmHg. The structure was supported by its NMR spectroscopic data.

### EXAMPLE 30

### Preparation of 2,2-Bis(Trifluoromethyl)-4,5-Di(2-Cyanoethyl)-1,3-Dioxolane

A reaction mixture comprised of Ni(TPP)₄ (3.4 g), TTP (2.1 g), toluene (25 ml), 25% EtAlCl₂/toluene (1.8 M, 2.0 ml) and the compound of Example 29 (24 g, 0.092 mole) was treated at 60°C with a 50% HCN/toluene solution at 1.5 ml/hr for 4.5 hrs and then 0.5 ml/hr until a gas chromatography showed complete conversion to the dinitrile. The mixture was cooled and a white solid was formed. The solid was collected by filtration and was dissolved in acetonitrile, washed with hexanes, after drying over magnesium sulfate, the solvent was removed and the residue was recrystallized from methanol. 10 g of the desired product was obtained. Mp. 58 to 59.5°C. H-1 NMR (CDCl₃): 2.06 (s, br, 4H), 2.64 (s, Br, 4H), 4.20, 4.70 (2s, br, 2H); F-19 NMR (CDCl₃): -81.0 (s, br) and -80.2, -80.4 (2s, br).

### EXAMPLE 31

### Preparation of 2,2-Bis(Trifluoromethyl)-4,5Di(3-Aminopropyl)-1,3-Dioxolane

The compound of Example 29 (9.72 g, 0.031 mole) was mixed with tetrahydrofuran (60 ml), Raney-Cobalt (2.8 g) and ammonia (12 g, 0.706 mole) in a 360 ml Hastelloy-C Shaker tube. The tube was pressurized with hydrogen to 500 psi at room temperature. The tube was then heated at 110°C for 18 hrs while the hydrogen pressure was adjusted to 1500 psi under the reaction process. The product mixture was filtered first, then was distilled to give the desired product 4.3 g as a clear, colorless liquid. Bp 85°C/0.02 mmHg. H-1 NMR (CDCl₃) : 3.98 (s, br, 2H), 2.76 (t, J = 6 Hz, 4H), 1.80-1.50 (m, 8H), 1.30 (s, br, 4H); F-19 NMR (CDCl₃): -81.1 (s, 6F).

## Claims

1. Dioxolanes of the formula 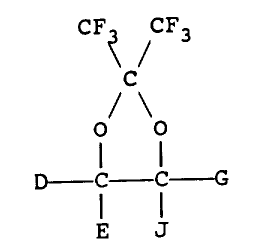 wherein D, E, G and J are:

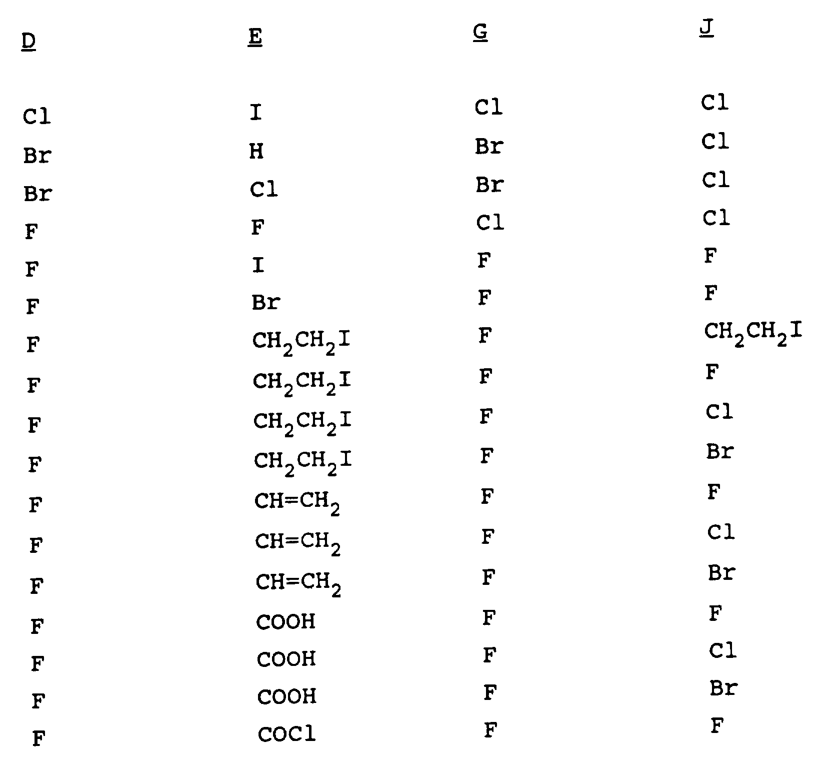

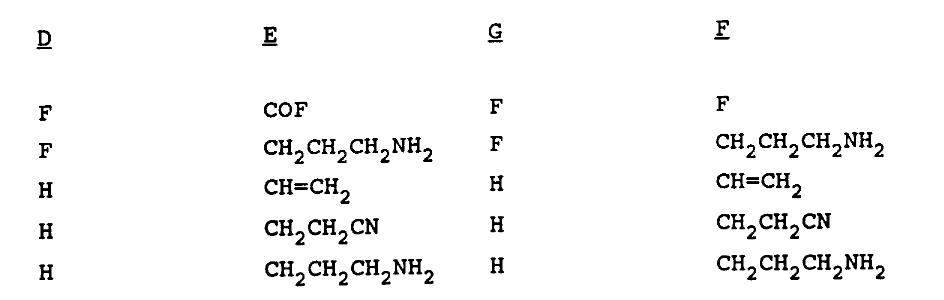

2. Copolymers of tetrafluoroethylene with at least one weight percent of a dioxolane having the formula recited in claim 1 wherein E and J are CH=CH₂ and D and G are F.

3. Copolymers of tetrafluoroethylene with at least one weight percent of the dioxolane of claim 1 wherein E and J are CH=CH₂ and D and G are H.

4. Copolymers of tetrafluoroethylene with at least one weight percent of the dioxolanes of claim 1 wherein D, E, G and J are:
| D | E | G | J |
|---|---|---|---|
| F | CH=CH₂ | F | F |
| F | CH=CH₂ | F | Cl |
| F | CH=CH₂ | F | Br |

## Patentansprüche

1. Dioxolane der Formel 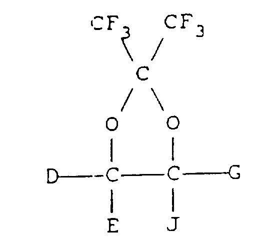 worin D, E, G und J 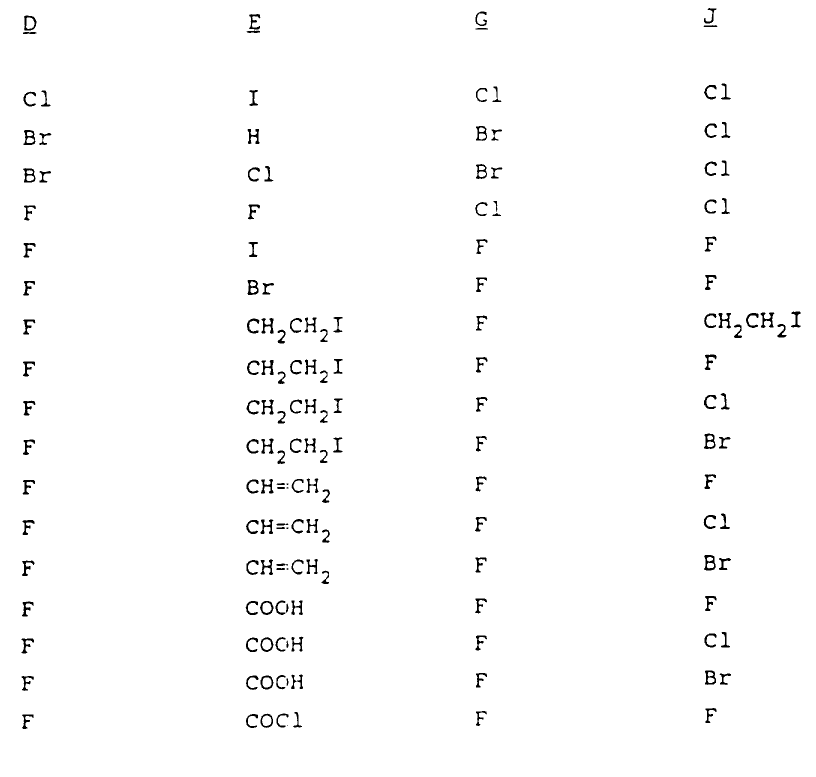 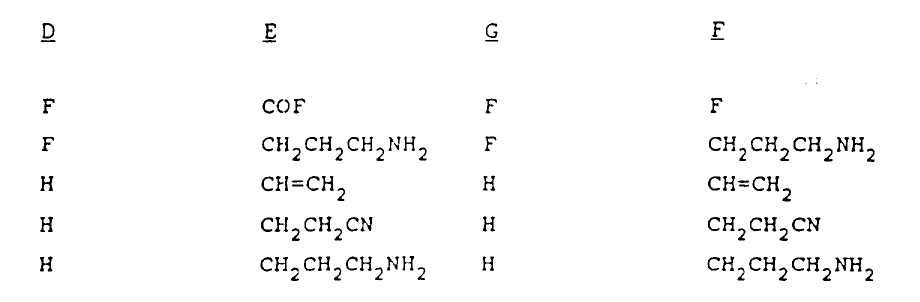 sind.

2. Copolymere des Tetrafluorethylens mit wenigstens 1 Gew.-% eines Dioxolans, welches die in Anspruch 1 aufgeführte Formel hat, worin E und J CH=CH₂ und D und G F sind.

3. Copolymere des Tetrafluorethylens mit wenigstens 1 Gew.-% des Dioxolans gemäß Anspruch 1, worin E und J CH=CH₂ und D und G H sind.

4. Copolymere des Tetrafluorethylens mit wenigstens 1 Gew.-% der Dioxolane gemäß Anspruch 1, worin D, E, G und J
| D | E | G | J |
|---|---|---|---|
| F | CH=CH₂ | F | F |
| F | CH=CH₂ | F | Cl |
| F | CH=CH₂ | F | Br |
sind.

## Revendications

1. Dioxolannes de la formule 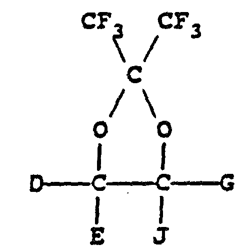 où D, E, G et J sont :
| D | E | G | J |
|---|---|---|---|
| Cl | I | Cl | Cl |
| Br | H | Br | Cl |
| Br | Cl | Br | Cl |
| F | F | Cl | Cl |
| F | I | F | F |
| F | Br | F | F |
| F | CH₂CH₂I | F | CH₂CH₂I |
| F | CH₂CH₂I | F | F |
| F | CH₂CH₂I | F | Cl |
| F | CH₂CH₂I | F | Br |
| F | CH=CH₂ | F | F |
| F | CH=CH₂ | F | Cl |
| F | CH=CH₂ | F | Br |
| F | COOH | F | F |
| F | COOH | F | Cl |
| F | COOH | F | Br |
| F | COCl | F | F |
| D | E | G | F |
|---|---|---|---|
| F | COF | F | F |
| F | CH₂CH₂CH₂NH₂ | F | CH₂CH₂CH₂NH₂ |
| H | CH=CH₂ | H | CH=CH₂ |
| H | CH₂CH₂CN | H | CH₂CH₂CN |
| H | CH₂CH₂CH₂NH₂ | H | CH₂CH₂CH₂NH₂ |

2. Copolymères de tétrafluoroéthylène avec au moins un pour cent en poids d'un dioxolanne ayant la formule citée dans la revendication 1 où E et J sont CH=CH₂ et D et G sont F.

3. Copolymères de tétrafluoroéthylène avec au moins un pour cent en poids du dioxolanne de la revendication 1 où E et J sont CH=CH₂ et D et G sont H.

4. Copolymères de tétrafluoroéthylène avec au moins un pour cent en poids des dioxolannes de la revendication 1 où D, E, G et J sont :
| D | E | G | J |
|---|---|---|---|
| F | CH=CH₂ | F | F |
| F | CH=CH₂ | F | Cl |
| F | CH=CH₂ | F | Br |
